# EUROPEAN PATENT APPLICATION

(11) **EP 4 847 491 A1**
(43) Date of publication of application: **02.12.2026**
(21) Application number: 25186795.8
(22) Date of filing: 01.07.2025
(51) Int. Cl.: C07D 251/60, C07D 251/62, B01J 19/00

(54) **IMPROVED PROCESS FOR THE PURIFICATION OF A MELAMINE SOLUTION AND RELATIVE APPARATUS**

(30) Priority: 30.05.2025 IT 202500012697
(71) Applicant: EUROTECNICA CONTRACTORS AND ENGINEERS S.p.A., 20159 Milano (IT)
(72) Inventor: DE AMICIS, Alberto, 20097 San Donato Milanese (MI) (IT); DI RUOCCO, Giuseppe, 23900 Lecco (LC) (IT); SANTUCCI, Roberto, 21050 Gorla Maggiore (VA) (IT); SERRAFERO, Alberto Carlo, 20098 San Giuliano Milanese (MI) (IT); PETRILLO, Francesca, 20161 Milano (IT)
(74) Representative: Bird & Bird Società tra Avvocati S.r.l.

(57) **Abstract**

An improved process for the purification of a melamine solution and the relative apparatus are described.

## Description

The present invention relates to an improved process for the purification of a melamine solution and to the relative apparatus.

In particular, the present invention relates to a process for the purification of melamine produced by synthesis from urea with high-pressure processes that operate in the liquid phase with the formation of a gaseous phase mainly composed of ammonia and carbon dioxide and a liquid phase composed of raw melamine and more specifically to a process that allows the reconversion into melamine of a part of the impurities formed in the synthesis or during the subsequent manipulations of the melamine.

It is known, in fact, that in the synthesis of melamine from urea, intermediate reaction by-products are formed which affect the purity of the melamine. The most important of these intermediate reaction products are ammeline and ammelide, usually indicated as oxyaminotriazines (OATs), whose presence in the product in quantities greater than 500-1000 ppm is harmful for the subsequent formation of melamine-formaldehyde resins.

In addition to OATs, during the synthesis reaction and downstream of the same, under particular conditions of reduced partial pressure of ammonia, another category of by-products is formed by the condensation reaction of several melamine molecules between each other and the consequent release of one or more ammonia molecules. These by-products are known under the collective name of polycondensates. Examples of polycondensates are melam and melem, to name only the most common, characterized by the following molecular structures:

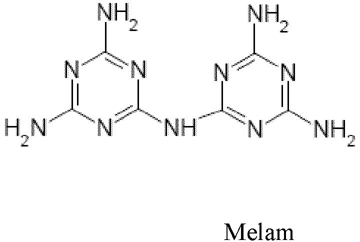

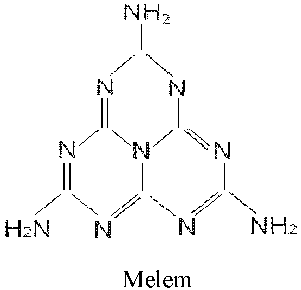

The lower the partial pressure of ammonia, the higher the temperature and the longer the melamine remains under the reaction conditions, typically a pressure higher than 70 bar and temperatures around 380°C, the higher the formation of polycondensates will be.

Like OATs, these products are also harmful for the subsequent use of melamine in the formation of resins by condensation with formaldehyde. A level of polycondensates lower than 1000 ppm in the finished product represents a limit of acceptability of the product.

The reactions that lead to the formation of polycondensates are reversible. Melamine contaminated with polycondensates can therefore be purified by subjecting it to the prolonged action of ammonia, under suitable conditions of temperature and partial pressure. Through the action of ammonia, not only are the polycondensates eliminated, but, as these are transformed back into melamine, a recovery of the overall yield of the process is also obtained.

The operating conditions that allow this objective to be achieved, however, are economically very demanding, as they require the treatment of the molten melamine at partial ammonia pressures above 250 bar, preferably above 400 bar, to obtain a melamine having an acceptable purity.

The conditions for the transformation of polycondensates in an aqueous ammonia environment are, on the other hand, milder and more economically favourable.

Most of the industrially operating melamine synthesis processes involve the recovery in an aqueous medium of the raw melamine exiting from the synthesis reactor. The process consists of feeding the reaction product, consisting of a liquid phase, raw melamine, and a gaseous phase mainly composed of NH₃ and CO₂, into a special contact apparatus (Quench Tower, or simply Quench) where the reaction product is cooled in the presence of water in which the raw melamine dissolves, together with part of the gaseous phase. At the same time, the pressure is reduced from the reaction pressure (above 70 bar) to a pressure normally lower than 25 bar, corresponding to a thermodynamic equilibrium temperature lower than 165°C, to limit corrosion phenomena.

Following this operation, a gaseous phase containing NH₃, CO₂ and steam and a liquid aqueous phase are formed which contains in solution, in addition to the NH₃ and CO₂ corresponding to thermodynamic equilibrium, melamine, OATs, polycondensates, unreacted urea and other minor impurities. The melamine content in the liquid phase can range from 5 to 15% by weight, whereas the OATs are present in a quantity of 0.1-0.4% by weight in the solution. In particular, the polycondensates are present in the aqueous solution in a ratio that ranges from 1:30 to 1:60 by weight with respect to melamine and their physical state (solution or suspension) depends on the particular temperature conditions and ammonia concentration adopted.

By subjecting the solution/suspension exiting from the Quench to the action of ammonia in a specific device into which ammonia is fed with the aim of generating an ammonia solution (NH₃ Digester or simply Ammonolyzer) the polycondensates are transformed into melamine. This transformation, however, proceeds at a rate that is lower, the higher the concentration of CO₂ in the liquid phase, thus requiring very lengthy contact times to reach a degree of transformation of the polycondensates compatible with the specific purity requirements of the melamine.

For this purpose, in industrial practice, the above-mentioned solution/suspension, with or without the addition of further ammonia, is subjected to a polycondensate reconversion treatment at a temperature generally lower than 165°C for a sufficient time ranging from one to several hours. This operation requires considerable volumes for the treatment, which involves, given the temperature and pressure conditions and given the nature of the material subjected to the treatment itself, the use of special stainless steels, very thick sheets and therefore high costs. During this treatment, there is also an undesired loss of product due to the simultaneous hydrolysis reaction of the melamine with water with the formation of OATs.

An improved process for the reconversion of polycondensates present as impurities in melamine produced by the pyrolysis of urea is described in US Patent 6,774,234 (US'234). The process involves dissolving the raw melamine in an aqueous environment in the Quench apparatus, subsequently removing the CO₂ to a concentration of less than 0.5% by weight and then reacting with ammonia (in the Ammonolyzer) added at a rate of 5-25% by weight, at a temperature ranging from 100 to 250°C for a few minutes. From the above-mentioned ammonolysis treatment, a solution is recovered having a concentration of polycondensates of less than 1000 ppm, typically less than 100 ppm, from which the melamine can then be crystallized.

EP2119711A1 (EP'711) describes a process capable of purifying melamine from polycondensates, which however requires the separation of the CO₂ by means of a steam stripping step. The decarbonated solution exiting from this CO₂ stripping step is fed into a special apparatus (Ammonolyzer) and ammonia is added, in which the polycondensates are reconverted into melamine by means of an ammonolysis reaction. The solution exiting from this step, however, has a higher content of OATs than the initial content produced in the reactor and fed to the Quench apparatus together with the raw melamine; this increase is due to the hydrolysis of melamine with water and is proportional to the temperature and residence time under the operating conditions of the Quench, Stripping and Ammonolysis steps. In the ammonolysis step, a conversion of the polycondensates into melamine of approximately 70% took place. Also in this process, as for the process described in US 6,774,234 (US'234), three different steps are provided for obtaining an ammonia solution of melamine, substantially free of CO₂, to be fed to the subsequent purification steps with activated carbons. The three steps are:
~ Quench with water
~ Steam stripping
~ Ammonolysis (digestion) with an ammonia solution.

The objective of the present invention is therefore to identify a process for the purification of melamine that overcomes the drawbacks previously specified, obtaining the almost complete decomposition of the polycondensates into melamine and, at the same time, minimizing the formation of new OATs.

The present invention therefore relates to an improved process for the purification of a melamine solution obtained by synthesis from urea in a high-pressure synthesis section with reactor and post-reactor, which comprises or consists of the following steps:
a) bringing into an aqueous ammonia solution, at a temperature ranging from 110 to 180°C, preferably from 130 to 140°C, a liquid phase of raw melamine, comprising polycondensates, other by-products of the synthesis reaction such as OATs, and NH₃, substantially free of CO₂, coming from the outlet of the synthesis section, obtaining a solution/suspension in which the insoluble products are dispersed; this solution/suspension containing ammonia in a quantity ranging from 1 and 20%, preferably from 3 to 15% by weight, with respect to the total weight of the solution/suspension,
b) putting the solution exiting from step a) in contact with a solid catalyst or solid catalytic carrier, under the same conditions as step a).

At the end of step b) the concentration of polycondensates in the melamine solution is reduced to values lower than 100 ppm by weight and the formation of OATs during the steps described is limited proportionally to the shorter residence time in both steps a) and b).

Liquid phase substantially free of CO₂ in this patent application means that the maximum quantity of CO₂ is less than the value of 0.5% by weight, more preferably less than 0.1% by weight.

The synthesis section with reactor and post-reactor can consist of two operating units, i.e. a reactor and post-reactor, or it can consist of a single operating unit in which reactor and post-reactor are integrated.

Step a) is carried out in a single dissolution apparatus (Dissolver), wherein the Quench and Ammonolysis steps provided in separate units in the processes of the state of the art, take place simultaneously: in step a) of the process according to the present invention, the dissolution of raw melamine coming from the synthesis section takes place, in fact, directly in an ammonia solution under the conditions described above.

The liquid phase fed to step a) is substantially free of CO₂ and urea.

The solid catalyst or solid catalytic carrier used in step b) is preferably activated carbon with a specific surface area ranging from 1000 to 2000 m²/g, more preferably from 1200 to 1500 m²/g, even more preferably equal to 1250 ₘ²/_{g}.

In particular, the total residence time of the melamine in the two steps, a) and b) of the process according to the present invention is less than 30 minutes.

A first advantage of the process according to the present invention is linked to the purification of a liquid stream of melamine exiting from the outlet of the synthesis section, i.e. coming out of the post-reactor: in the present invention, in fact, the first purification step (in the Dissolver) is fed exclusively with the liquid phase exiting from the post-reactor, consisting of the reaction product, i.e. the raw melamine.

The gaseous phase present in the synthesis section has already been separated from said liquid phase in the reactor and in the post-reactor and the liquid phase, exiting from the post-reactor in which a substantially complete conversion of urea to give melamine has taken place, substantially contains neither urea nor CO₂. The liquid phase that feeds the purification phase therefore contains melamine, OATs, polycondensates and ammonia.

Considering that the reconversion process of polycondensates into melamine is slower, the greater the quantity of CO₂ present in the liquid phase, in the processes of the state of the art, a stripping treatment is inevitably necessary for removing the CO₂ contained in the aqueous solution exiting from the Quench step, and this further treatment leads to an increase in the residence times, as the Quench step with water and the Ammonolysis step with ammonia necessarily take place in two distinct steps conducted in separate units, respectively before and after the CO₂ stripping step, thus favouring the formation of OATs (another undesired by-product such as polycondensates); the process according to the present invention, wherein the purification is carried out on the liquid phase comprising raw melamine substantially free of CO₂ exiting from the post-reactor, has the significant advantage of requiring much shorter residence times.

Compared to the state of the art, in fact, as the present invention does not require the stripping of CO₂ with steam, the Quench and Ammonolysis steps can be carried out directly in a single unit, thus reducing the residence times thanks to the reduction in the volumes, under the same process conditions, in terms of ammonia concentration, temperature and pressure, necessary for the subsequent purification steps.

This eliminates the distinct Quench step with water and Stripping step with steam: the liquid phase of raw melamine coming from the reaction section is dissolved with ammoniacal water directly in a single step a) with a residence time in the dissolution apparatus (Dissolver) sufficient for the transformation of the polycondensates into melamine, incorporating the Quench and Ammonolysis functions in this dissolver.

A further advantage of the process according to the present invention also consists in the reduction of the times of step b): thanks to the use of a particular catalyst, in fact, the process according to the present invention allows the polycondensates to be almost completely converted into melamine, with reduced residence times and therefore with reduced volumes, while operating under process conditions similar to those of the state of the art.

The quantity of ammonia can in fact be reduced as there is no CO₂ present: a reduced quantity of ammonia is therefore sufficient for shifting the reversible reaction of the polycondensates towards the formation of melamine and, thanks to the use of a catalyst with specific characteristics capable of promoting the formation of melamine from polycondensates, this reaction takes place with a reduced residence time which also allows for a reduction in the formation of OATs due to the hydrolysis of melamine.

Thanks to step a) for dissolution in aqueous ammonia solution and to the use of a catalyst in step b) with specific characteristics, and therefore thanks to a reduction in the residence times, the solution according to the present invention allows the formation and therefore the quantity of OATs to be reduced as much as possible, also allowing the volumes of the purification section to be reduced thanks to the use of a single apparatus that combines the Quench and Ammonolysis functions.

Reducing the quantity of OATs also allows the recycling of a greater quantity of mother liquor coming from the separation step of the melamine crystal in the purification sections downstream.

Furthermore, recycling a greater quantity of mother liquor allows a reduction in the energy consumption relating to the subsequent mother liquor treatment steps for recovering ammonia and decomposing the melamine.

The process according to the present invention is therefore an optimized purification process which, thanks to the treatment of a raw melamine substantially free of CO₂ and thanks to the use of a catalyst with specific characteristics, also allows the use of a limited quantity of NH₃, which is sufficient however to keep the OATs in solution, as the latter are in turn present in smaller quantities thanks, specifically, to the reduction in the residence times, due to the reduction in the volumes, having combined the Quench step with water and the digestion step with ammonia (Ammonolysis) and having completely removed the CO₂ stripping step.

Not only have the residence times in the dissolution step a) been reduced, also thanks to the increased reaction rate of the polycondensates in the absence of CO₂ in the melamine solution, but also the quantity of catalyst in step b) is optimized, again thanks to the fact that the conversion reaction of the polycondensates is faster, as CO₂ is substantially absent and an appropriate catalyst is used.

The solution according to the present invention thus allows a reduction in the quantity of solid catalyst required, preferably activated carbon with specific characteristics suitable for promoting the reconversion of the polycondensates into melamine as much as possible, ensuring close contact of the solution with the catalyst.

In reducing the quantity of activated carbon used in step b), by using an activated carbon with appropriate specifications, the residence times are consequently reduced thanks to the reduced volume of the section, making the formation of OATs more difficult.

The quantity of solid catalyst or catalytic carrier, i.e. activated carbon with a specific surface area ranging from 1000 to 2000 m²/g, more preferably from 1200 to 1500 m²/g, even more preferably equal to 1250 m²/g, used in step b), depends on the catalysis space velocity; this velocity is defined as the ratio between the volumetric flow-rate (m³/h) of the reagent solution and the volume of the catalyst (m³), and is expressed in h⁻¹.

This velocity in step b) of the process according to the present invention is within the range of 5-8 h⁻¹, preferably from 6 to 7 h⁻¹, and surprisingly allows an almost complete conversion of the polycondensates to be obtained, while minimizing the formation of OATs.

The Applicant has therefore surprisingly found that the drawbacks indicated by the state of the art can be overcome by subjecting the liquid phase of raw melamine to a digestion treatment with NH₃, under much milder conditions than those envisaged by US'234, and in significantly shorter times than the process described in EP'711, eliminating the CO₂ stripping step and combining the Quench and Ammonolysis phases in step a), said dissolution step a) being followed by a finishing step on a solid catalytic carrier with specific technical characteristics, operating under unchanged conditions of temperature and concentration of NH₃.

As indicated above, the solid catalyst or solid catalytic carrier used in step b) is preferably activated carbon with a specific surface area within the range of 1000 to 2000 m²/g, more preferably from 1200 to 1500 m²/g, even more preferably equal to 1250 m²/g. It has been found that this specific surface-area range is optimal for the catalyst to be effective under the conditions indicated in the process according to the present invention, and together with a catalysis space velocity within the range indicated (5-8 h⁻¹), it allows a reduction in the residence times to be obtained, thanks to the reduction in the catalyst volume, minimizing the formation of OATs while almost completely converting the polycondensates (below the analytical limit).

A lower space velocity would allow the complete conversion of the polycondensates to the detriment of an increase in OATs following an increase in the quantity (volume) of catalyst (or the decrease in flow-rates).

In particular, it has been observed that the succession of these two steps (dissolution and finishing), carried out under the appropriate conditions and by using an activated carbon having a specific surface as indicated above, makes it possible to achieve the reduction of polycondensates in the solution/suspension to values lower than 100 ppm, also thanks to a space velocity that allows operating at lower temperatures, with reduced volumes and residence times compared to the processes known in the state of the art, while also reducing the formation of OATs. From the solution thus purified, high-purity melamine can be obtained, by crystallization and subsequent drying, with appreciable advantages in terms of consumption and costs.

Step a) of the process according to the present invention consists in bringing into solution, i.e. dissolving in water and ammonia, a liquid phase of raw melamine coming from the synthesis section, obtaining a melamine concentration ranging from 5% to 19% by weight, preferably from 7% to 15% by weight and more preferably from 9% to 11% by weight, with respect to the weight of the solution/suspension. The melamine contains impurities of polycondensates and other by-products of the synthesis reaction that can remain in suspension as they are scarcely soluble. The ammonia solution of melamine, exiting from step a), has an ammonia concentration ranging from 1 to 20%, preferably from 3 to 15% by weight, with respect to the total weight of the solution/suspension, at a temperature of 110-180°C, preferably at a temperature of 130-140°C and at the corresponding equilibrium pressure.

The dissolution step a) is kept under these conditions, for a time of 5 to 15 minutes, preferably less than 10 minutes.

The polycondensates present in the original solution/suspension are converted into melamine for over 75% of their original value, thanks to the absence of CO₂ - and therefore without the need for resorting to a CO₂ stripping step - and thanks to ammonia concentrations that favour the conversion of the polycondensates, with reduced residence times that limit the formation of OATs. The increase in the conversion of the polycondensates present in the solution/suspension is also guaranteed by the use of a catalyst with a certain specific surface.

A fundamental step of the process according to the present invention is step b), i.e. the finishing treatment in which the solution exiting from step a) is put in contact with the solid catalyst under the same conditions as step a). Step b) is preferably carried out with a solid catalyst consisting of an activated carbon bed with specific technical characteristics. This finishing step, although aimed at completing the transformation of the polycondensates remaining from the dissolution step a), is also capable of removing any other impurities, soluble and/or insoluble, present in the solution with an improvement in the final colour of the product.

In particular, the total residence time of the melamine in the two steps of the process according to the present invention is less than 30 minutes.

Unlike the process according to the state of the art described in EP'711, in the process according to the present invention it is also possible to have a lower volume of the catalytic bed, both because the polycondensates exiting from the dissolution step a) are in a lower quantity due to a practically negligible CO₂ content in the solution/suspension (lower than the value of 0.5% by weight obtained in the state of the art by steam stripping), and also due to the use of a catalyst with a specific surface area such as to guarantee the almost total reconversion of the polycondensates to melamine.

The catalyst used in step b) has a preferred specific surface of about 1250 m²/g which, together with a space velocity within the range of 5-8 h⁻¹, preferably ranging from 6 to 7 h⁻¹, allows the polycondensates to be almost completely converted (reducing their content in the solution below the analytical limit) and at the same time by reducing the residence times, thanks to the reduction in the volumes, it minimizes the formation of OATs. A higher space velocity would not guarantee a complete conversion of the polycondensates, a velocity lower than 5 h⁻¹, on the other hand, would require larger catalyst volumes or lower volumetric flow-rates of the solution, consequently leading to a significant increase in the residence time and therefore an increase in the formation of OATs. This finishing step b) is, as already mentioned, also fundamental for the filtering effect of the insoluble particles present in the solution with an improvement in the quality of the final melamine.

The total transformation of the polycondensates on the solid catalyst, in particular on the activated carbon, can then be preceded by a mechanical filtration step with the aim of retaining any further insoluble impurities present in the solution exiting from step a). The mechanical filtration steps helps to avoid or, at least, slow down the possible deactivation of the solid catalyst bed, preferably activated carbon, due to pure mechanical clogging.

The process according to the present invention can also comprise a second mechanical filtration step of the solution containing melamine, downstream of the finishing treatment on the activated carbon bed, in order to retain and remove any solid impurities still present, including catalyst residues.

The present invention further relates to an apparatus for carrying out the purification process of a melamine solution coming from the synthesis section, said apparatus being composed of a purification section comprising:
~ a column for the treatment of raw melamine containing polycondensate impurities and other by-products of the melamine synthesis reaction, with an aqueous ammonia solution (Dissolver);
~ a finishing unit comprising a catalytic bed for completing the transformation of the residual polycondensates present in the raw melamine solution/suspension exiting from the column for treatment with ammonia, wherein said catalytic bed consists of a solid catalyst which is activated carbon with a specific surface area varying within the range of 1000 to 2000 m²/g, more preferably from 1200 to 1500 m²/g, even more preferably equal to 1250 m²/g.

Said finishing unit is preferably composed of a single block which comprises in sequence a first filtration element, the catalytic bed for completing the transformation of the residual polycondensates present in the solution/suspension of raw melamine exiting from the column for treatment with ammonia and a second filtration element or alternatively it can be composed of three distinct elements which consist of a first filtration element, the catalytic bed and the second filtration element in sequence.

The finishing unit produced in a single block can also comprise an inlet for the solution coming from the column for the treatment with ammonia (dissolver) that bypasses the first filtration element and enters directly into the catalytic bed and an outlet from the catalytic bed that bypasses the second filtration element. This embodiment is used when the process according to the present invention does not include the filtration steps that precede and follow step b).

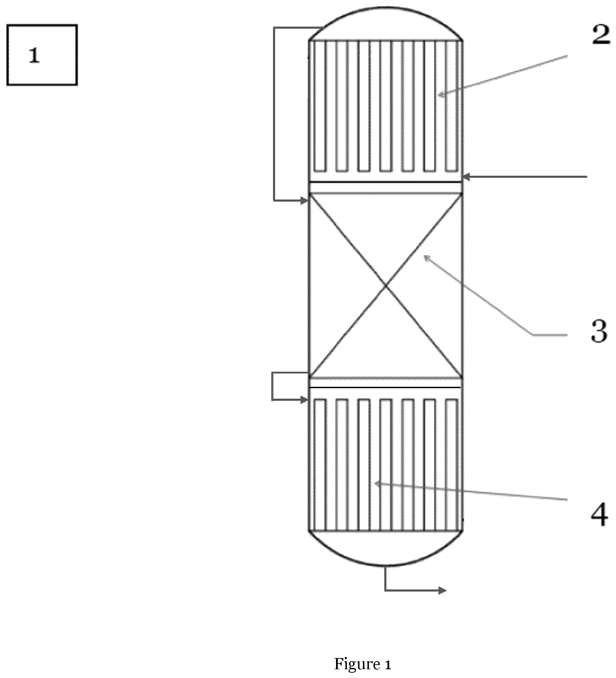

An embodiment of the finishing unit of the apparatus according to the present invention is shown in Figure 1, where 1 indicates the finishing unit, 2 the first filtration element, 3 the catalytic bed and 4 the second filtration element.

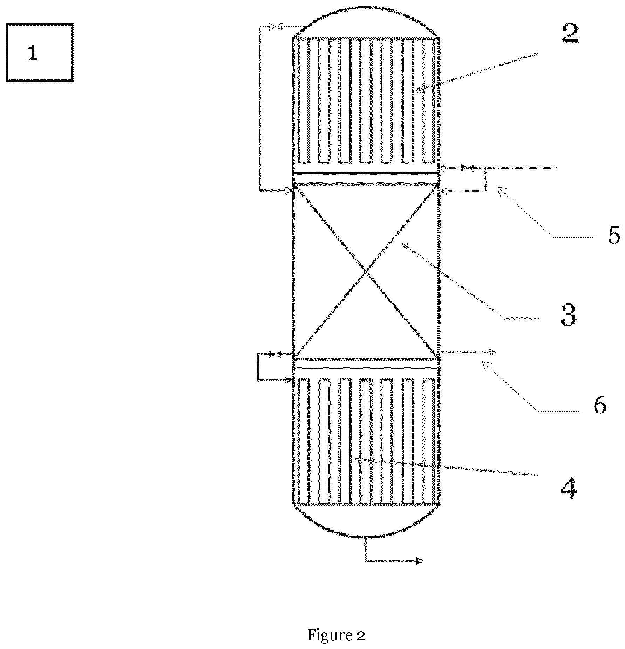

A second embodiment of the finishing unit of the apparatus according to the present invention is shown in Figure 2, where 1 indicates the finishing unit, 2 the first filtration element, 3 the catalytic bed and 4 the second filtration element. 5 and 6 indicate the two by-passes that allow the aqueous ammonia solution/suspension to bypass the first filtration element and enter directly into the catalytic bed and to exit from the catalytic bed bypassing the second filtration element.

In the embodiment of Figure 1, the finishing unit is composed of a single block that optimizes the purification section compared to the solutions of the state of the art, in which the above-mentioned three elements (first filtration element, catalytic bed and second filtration element) were three distinct devices with functional connections (for example pipes or other connection elements). The single block according to the present invention, on the other hand, allows the presence of functional connections to be minimized, that also lead to an increase in the volumes of the equipment and consequently an increase in residence times, favouring the formation of OATs.

The column for the treatment with ammonia of the solution/suspension of raw melamine containing impurities of polycondensates and other by-products of the synthesis reaction (not shown in figure 1) can be composed of a conventional tower having a height/diameter ratio greater than 2.

The catalytic bed with which the solution/suspension of raw melamine is put in contact in the finishing step of the purification process is an activated carbon bed with the specific characteristics indicated above.

Said first filtration element, in a preferred embodiment of the filtration unit, consists of a mechanical filtration means for removing the impurities remaining in suspension in the solution exiting from the ammonia treatment column. Said means, for example a bed of inert solid material, is positioned upstream of the catalytic bed and is functionally connected to the column from which it receives the ammonia-treated solution. The mechanical filtration of the solution, in addition to purifying the melamine by retaining the insoluble solid impurities, helps to avoid clogging of the catalytic bed and, therefore, to delay its deactivation.

Said second filter element, in a preferred embodiment of the filter unit, consists of further mechanical separation means (guard filter) downstream of the catalytic bed from which they receive the solution containing the melamine in order to retain any solid impurities present after the treatment on the catalytic bed, including any catalyst residues.

The orientation of the first and second filter elements is not a parameter that influences the performance of the equipment; therefore, elements 2 and 4 shown in figure 1 and figure 2, can be oriented in any direction that allows their functionality.

Compared to the processes known in the state of the art, the process according to the present invention and the relative apparatus for implementing it allow the polycondensate impurities to be eliminated from the ammonia solution containing the melamine to be crystallized, in a satisfactorily short time (less than 30 minutes, to be compared with a minimum residence time of the state of the art of 40-50 minutes), allowing a greater recycling of the mother liquors, proportionally to the lower quantity of OATs that have been formed, with a consequent increased saving of steam for the recovery of the same in the subsequent steps of the melamine production process in which the purification process according to the present invention is inserted.

The apparatus for carrying out the purification process of a melamine solution according to the present invention can be inserted into a melamine production plant under construction or into an existing plant.

The conditions of the purification process of the melamine solution, which are much milder than those of the known purification processes, also lead to an increase in the overall yield of the melamine production process, as they allow operating with residence times that are much shorter than those of the current state of the art. Under the optimal treatment conditions, the formation of OATs by hydrolysis of melamine is in fact significantly reduced.

The temperature and pressure conditions of step a) are optimized within the range of 25-40 bar and 110-180°C depending on the concentration of NH₃ used: a higher concentration of NH₃ favours the formation of melamine starting from polycondensates.

Some application examples are provided for a better illustration of the objectives and advantages of the present invention, which, however, in no way limit the scope of the claims.

### Example 1

3978 kg/h of a liquid phase of raw melamine having the following composition were taken from the outlet of the post-reactor:

| | |
|---|---|
| Melamine (100%) | 3843 kg/h |
| Polycondensates | 50 kg/h |
| Unreacted urea | absent |
| NH₃ (in solution) | 77 Kg/h |
| CO₂ (in solution) | absent. |

This liquid phase was fed directly to the Dissolution column (DISSOLVER) where it was put in contact with ammoniacal water generating a melamine solution having the following composition (by weight):

| | |
|---|---|
| H₂O | 80.30% |
| melamine | 9.5% |
| NH₃ | 10% |
| Polycondensates | 0.05% (500 ppm) |
| OATs | 0.15% (1500 ppm). |

The solution was kept at a temperature of 160°C and a pressure of 25 bar and was left to rest inside the column for 10 minutes. The solution was then fed to an apparatus according to the present invention comprising in succession: a finishing unit having, in sequence, a mechanical filtration device, an activated carbon bed and finally a guard filter. The first mechanical filtration, the finishing treatment on the activated carbon bed (with a surface area equal to 1250 m²/g and a volume of approximately 7.5 m³, corresponding to a space velocity of 6 h⁻¹) and the filtration by means of the guard filter were carried out by maintaining the solution containing melamine under the same temperature and ammonia concentration conditions as the Dissolution column. The finishing steps on activated carbon and filtration involved keeping the melamine under the above conditions for a further 15 minutes (10 minutes for the section with activated carbon and the remaining for filtration), for a total residence time of the melamine in the two steps a)-b) of 25 minutes.

The solution exiting from the finishing unit has the following composition:

| | |
|---|---|
| H₂O | 80.279% |
| melamine | 9.5% |
| NH₃ | 10% |
| Polycondensates | 0.001% (10 ppm) |
| OATs | 0.22%. (2200 ppm) |

The residence times are such that the quantity of OATs does not exceed 2200 ppm in the solution exiting from the finishing unit, a solution that is then fed to the crystallization step of the melamine production process.

At the outlet of the finishing unit, the substantial total disappearance of the polycondensates is observed, their concentration being below the analytical limit of the detection method used (Ultraviolet spectrophotometry) and therefore below 10 ppm.

The colour of the melamine crystallized and dried according to the methods known in the art is lower than the value of 10 according to the APHA scale.

### Example 2 (comparative)

Example 1 was repeated under the same conditions with the exception of the type of solid catalyst in the finishing step. An activated carbon catalytic bed with a specific surface area of 500 m²/g was in fact used for treating an equal solution exiting from the Dissolver section with the following composition:

| | |
|---|---|
| H₂O | 80.30% |
| melamine | 9.5% |
| NH₃ | 10% |
| Polycondensates | 0.05% (500 ppm) |
| OATs | 0.15% (1500 ppm). |

The solution exiting from the finishing unit, with the same residence times and space velocities of the catalytic bed as in Example 1, has the following composition:

| | |
|---|---|
| H₂O | 80.25% |
| Melamine | 9.5% |
| NH₃ | 10% |
| Polycondensates | 0.03% (300 ppm) |
| OATs | 0.22% (2200 ppm). |

At the outlet of the finishing unit, a smaller reduction in the quantity of polycondensates is observed compared to Example 1, due to the use of a catalytic bed which does not have an optimal specific surface for obtaining melamine free of polycondensates.

### Example 3 (comparative)

Example 1 was repeated using the same type of catalyst with a specific surface area of 1250 m²/g, but with a quantity of catalyst which is such as to have a space velocity higher than 8.5 h⁻¹ (reducing the catalyst volume by 30% gives a space velocity higher than 8.5 h⁻¹). The same solution exiting from the treatment section with water and NH₃ (Dissolver) was subjected to purification treatment with the following composition:

| | |
|---|---|
| H₂O | 80.30% |
| melamine | 9.5% |
| NH₃ | 10% |
| Polycondensates | 0.05% (500 ppm) |
| OATs | 0.15%.(1500 ppm) |

The solution exiting from the finishing unit has the following composition:

| | |
|---|---|
| H₂O | 80.27% |
| Melamine | 9.5% |
| NH₃ | 10% |
| Polycondensates | 0.03% (300 ppm) |
| OATs | 0.20% (2000 ppm). |

At the outlet of the finishing unit, a lower quantity of polycondensates is detected compared to Example 1, due to a high space velocity of the catalytic bed, while maintaining the same residence times, which are such as not to guarantee an optimal conversion of the polycondensates into melamine; their concentration is therefore equal to approximately 300 ppm, much higher than the 10 ppm detected for Example 1 according to the invention.

### Example 4 (comparative)

A two-phase stream exiting from the reactor outlet comprising both raw melamine and reaction off-gas is treated in a Quench with water for a residence time of 8 minutes. The solution exiting from the Quench and having the following composition (by weight):

| | |
|---|---|
| H₂O | 68.5% |
| Melamine | 10% |
| OATs | 0.3% (3000 ppm) |
| Polycondensates | 0.23% (2300 ppm) |
| Urea | 0.47% |
| NH₃ | 16.7% |
| CO₂ | 3.8% |

was subjected to steam stripping carried out at 5 bar and 160°C (bottom column values) for a total residence time in the column of approximately 10 minutes, thus obtaining a decarbonated solution having the following composition (again by weight):

| | |
|---|---|
| H₂O | 88.7 % |
| Melamine | 9.5% |
| OATs | 0.32% (3200 ppm) |
| Polycondensates | 0.24 % (2400 ppm) |
| Urea | 0.4% |
| NH₃ | 0.51% |
| CO₂ | 0.33% |

This solution was then subjected to a treatment with the addition of ammonia (Ammonolysis) under the conditions of 160°C and 25 bar; the ammonia solution containing melamine was left to stand inside the ammonolysis column for 10 minutes. The solution/suspension exiting from the Ammonolysis step was found to have the following composition:

| | |
|---|---|
| H₂O | 80.25% |
| melamine | 8.59% |
| OATs | 0.35% (3500 ppm) |
| Polycondensates | 0.15% (1500 ppm) |
| Urea | 0.36% |
| NH₃ | 10% |
| CO₂ | 0.30%. |

The first mechanical filtration, the finishing treatment on the activated carbon bed and the filtration by means of the guard filter were carried out by keeping the solution containing melamine under the same temperature and ammonia concentration conditions as the ammonia treatment column (ammonolysis). The finishing steps on activated carbon and filtration involved keeping the melamine under the above conditions for a further 15 minutes in total.

The solution exiting from the finishing unit has the following composition:

| | |
|---|---|
| H₂O | 80.25% |
| Melamine | 8.59% |
| OATs | 0.44% (4400 ppm) |
| Polycondensates | 0.06% (600 ppm) |
| Urea | 0.36% |
| NH₃ | 10% |
| CO₂ | 0.30%. |

The activated carbon used for the finishing step has a specific surface of 1250 m²/g, and is present in such a quantity that the catalysis space velocity is equal to 6 h⁻¹.

The increase in the quantity of OATs is evident, which has passed from 2900 ppm initial to 4400 ppm final compared to the solution. Therefore, due to the effect of the larger volumes and longer residence times compared to the process of Example 1 according to the invention, a loss of melamine due to the hydrolysis to OATs is evident.

The variations in the quantities of polycondensates and OATs indicated in the examples do not lead to significant variations in the analytical values of the melamine concentration which is two orders of magnitude higher than the quantities of impurities (detectable in ppm; example 0.44% by weight is equivalent to 4400 ppm).

The examples previously described demonstrate how the reduced residence times of the melamine in the steps indicated according to the present invention (Example 1 compared with comparative Example 4), allow the formation of OATs by hydrolysis of the melamine to be avoided, at the same time reducing the quantity of polycondensates below the analytical limit with a conversion of the same into melamine higher than 75%, a reduction in the residence times due to the removal of the CO₂ stripping section and thanks to the combination of the quench and ammonolysis steps in a single dissolution step in ammonia solution (Dissolver).

Furthermore, the results of Example 1 compared with comparative Example 2 indicate how the optimization of the specific surface of the solid catalyst according to the present invention also allows a reduction in the quantity of polycondensates to be obtained which is below the analytical limit with a conversion of the same into melamine higher than 75%.

A further comparison can be made by comparing Example 1 with comparative Example 3, in which the tests were conducted considering two different space velocities (therefore two different catalyst volumes), from which it emerges that with the process of the present invention an optimal space velocity was identified (not less than 6 h⁻¹ and not more than 8 h⁻¹) for a catalyst with the specific surface according to the present invention..

It is therefore evident how the optimization of all the parameters of the process according to the present invention, namely reduced residence times, together with a catalyst which is an activated carbon bed with specific surface characteristics and with a space velocity within the range indicated, allow a complete conversion of the polycondensates to be obtained, at the same time, minimizing the hydrolysis of the melamine to form OATs, obtaining an extremely high-quality melamine.

## Claims

1. An improved process for the purification of a melamine solution obtained by synthesis from urea in a high-pressure synthesis section with reactor and post-reactor, comprising or consisting of the following steps:
a) bringing into an aqueous ammonia solution, at a temperature ranging from 110 to 180°C, preferably from 130 to 140°C, a liquid phase of raw melamine, comprising polycondensates, other by-products of the synthesis reaction such as OATs, and NH₃, substantially free of CO₂, coming from the outlet of the synthesis section, obtaining a solution/suspension in which the insoluble products are dispersed; said solution/suspension containing ammonia in a quantity ranging from 1 to 20%, preferably from 3 to 15% by weight, with respect to the total weight of the solution/suspension,
b) putting the solution exiting from step a) in contact with a solid catalyst or solid catalytic carrier, under the same conditions as step a).

2. The process according to claim 1, wherein the solid catalyst or solid catalytic carrier is a bed of activated carbon.

3. The process according to claim 1 or 2, wherein the solid catalyst or solid catalytic carrier is activated carbon with a specific surface area within the range of 1000 to 2000 m²/g, more preferably from 1200 to 1500 m²/g, even more preferably equal to 1250 m²/g.

4. The process according to one or more of the previous claims, wherein the catalytic space velocity ranges from 5 to 8 h⁻¹, preferably from 6 to 7 h⁻¹.

5. The process according to one or more of the previous claims, wherein the total residence time of the melamine in steps a) to b) is less than 30 minutes.

6. The process according to one or more of the previous claims, comprising a first step for the mechanical filtration of the solution exiting from step a), before the treatment of step b).

7. The process according to one or more of the previous claims, comprising a second step for the mechanical filtration of the solution exiting from step b).

8. The process according to one or more of the previous claims **characterized in that** the solution exiting from step b) has a concentration of polycondensates lower than 100 ppm by weight.

9. An apparatus for effecting the purification process of a melamine solution coming from a melamine synthesis section starting from urea, consisting of a purification section comprising:
- an apparatus for carrying out the purification process of a melamine solution coming from the synthesis section, said apparatus being composed of a purification section comprising:
~ a column for the treatment of raw melamine containing polycondensate impurities and other by-products of the melamine synthesis reaction, with an aqueous ammonia solution;
~ a finishing unit comprising a catalytic bed for completing the transformation of the residual polycondensates present in the raw melamine solution/suspension exiting from the column for treatment with ammonia, wherein said catalytic bed consists of a solid catalyst which is activated carbon with a specific surface area within the range of 1000 to 2000 m²/g, more preferably from 1200 to 1500 m²/g, even more preferably equal to 1250 m²/g.

10. The apparatus according to claim 9, wherein the synthesis section with reactor and post-reactor consists of two operating units, or it consists of a single operating unit in which reactor and post-reactor are integrated.

11. The apparatus according to claim 9, wherein the finishing unit is composed of a single block which comprises in sequence a first filtration element, a catalytic bed for completing the transformation of the residual polycondensates present in the solution/suspension of raw melamine exiting from the column for treatment with ammonia and a second filtration element or, alternatively, it is composed of three distinct elements which consist of a first filtration element, the catalytic bed and the second filtration element in sequence.

12. The apparatus according to claim 11, wherein the finishing unit consisting of a single block provides an inlet for the solution coming from the ammonia treatment column which bypasses the first filter element and enters directly into the catalytic bed and an outlet from the catalytic bed which bypasses the second filter element.
